# EUROPEAN PATENT APPLICATION

(11) **EP 2 580 977 A1**
(43) Date of publication of application: **17.04.2013**
(21) Application number: 11837452.9
(22) Date of filing: 03.06.2011
(51) Int. Cl.: A43B 7/02, A43B 13/14

(54) **ELECTRIC HEATING SOLE**

(30) Priority: 04.11.2010 CN 201020590772 U
(71) Applicant: Ye, Ying, Dongguan, Guangdong 523071 (CN)
(72) Inventor: Ye, Ying, Dongguan, Guangdong 523071 (CN)
(74) Representative: Decamps, Alain René François
(86) International application number: PCT/CN2011/075267
(87) International publication number: WO 2012/058925

(57) **Abstract**

An electric heating sole comprises a sole main body (1). A heating layer (2) and a press type electric generator (3) are arranged in the sole main body (1). Heating elements are distributed in the heating layer (2). The heating layer (2) is connected with the press type electric generator (3). An intensive area (21) is arranged on the heating layer (2); the distribution density of the heating elements in the intensive area (21) is higher than that of the heating elements at other area of the heating layer (2); and the intensive area (21) is arranged at a position where the heating layer (2) corresponds to acupuncture points on the foot of a human body. The electric heating sole (1) has the advantages as follows: the comprehensive warming function is better; the special requirement that the important parts of the foot of the human body are kept warm can be met; the distribution of the produced heat is reasonable; the application range is wide; and the electric power can be supplied by the sole itself.

## Description

### TECHNICAL FIELD OF THE INVENTION

The invention relates to shoe technique field, especially to an electric heating sole.

### BACKGROUND OF THE INVENTION

Common sole is always made of cotton, fur and fleece, it has certain effect of keeping warmth but it cannot generate heat actively itself. It needs to be put inside the shoes and keeps warmth relaying on the sealing performance of shoes; however, when the temperature of external environment is much lower than that of human body, the feet will feel cold gradually and even be frozen. When the feet are frozen, temperature of other parts of human body will also be lower; the human life will be in danger if this lasts for long time. To avoid this problem, people need to wear the common warmth-keeping shoes; however, some people have feet easy to sweat, the warmth-keeping shoes will be wet after long time, so the shoes cannot provide good warmth-keeping function. Besides, the warmth-keeping shoes bring inconvenience to the wears, so it is hard to provide good warmth-keeping function.

To solve the problem, there is technique that converts the electric energy into the heat energy and supplies the heat energy to the sole to heat the sole and also bake the sole that might be wet. The Chinese patent CN201085130Y publishes an electric heating sole comprising a common sole; a resistance wire is arranged between the interlayer of the common sole and connected with a conducting wire which is connected with a power plug and switch. This electric heating shoe has a little warmth keeping effect, but it has disadvantage that it cannot meet the special requirement of important parts of human feet, for example, parts like arch of foot, forefoot and toe with more acupunctures directly related to visceral organs; these parts need more heat than other parts of feet of human body; if the heat is low, these parts cannot get effective heat supply; and if the heat is high, the energy is wasted and the human body will feel hot and dry, and the feet will sweat, which influence health of human body. Besides, power supply of the electric heating sole is battery, when the battery is exhausted, it is necessary to change or charge the battery, which is not convenient for use.

### SUMMARY OF THE INVENTION

Aiming at the disadvantages of the existing technique, the present invention claims an electric heating sole which has good comprehensive warming function, meets special requirement that the important parts of the foot of the human body are kept warm, and can provide electric power itself.

In order to realize the above goal, the invention adopts the following technical proposal.

An electric heating sole, comprising a sole main body, wherein a heating layer and a press type electric generator are arranged in the sole main body; the heating elements are distributed in the heating layer; the heating layer is connected with the press type electric generator; an intensive area is arranged on the heating layer; the distribution density of the heating elements in the intensive area is higher than that of the heating elements at other area of the heating layer; and the intensive area is arranged at a position where the heating layer corresponds to acupuncture points on the foot of a human body.

The intensive area is arranged at positions where the heating layer corresponds to arch of foot, forefoot and toe of the human body.

The press type electric generator includes a tread mechanism, one end of which is connected with a driving wheel that is connected with a micro electric generator; and output end of the micro electric generator is connected with the heating layer.

An electric storage device is arranged between the micro electric generator and the heating layer, and the electric storage device is separately connected with the micro electric generator and the heating layer.

One end of the tread mechanism is in swing connection with the sole main body through a connection axle which is sleeved with a spring; and the other end of the tread mechanism is provided with arc-shaped spline connected with the driving wheel.

The driving wheel comprises a first gearing and a second gearing that are coaxial and fixedly connected with each other; the first gearing is engaged with the arc-shaped spline of the tread mechanism and the second gearing is connected with the micro electric generator.

The micro electric generator comprises a third gearing that is engaged with the second gearing of the driving wheel, and the third gearing is fixedly connected with a ratchet wheel which is provided with a rotation disc on the top; electric generating elements are arranged in the rotation disc; both sides of the ratchet wheel are provided with detents, and inner wall of the rotation disc is provided with multiple detent blocks to coordinates with the multiple detents.

The electric storage device is connected with a control switch that is arranged outside of the sole main body.

The control switch comprises an ON shift, temperature adjustment shift and an OFF shift.

The sole main body is provided with an accommodation cavity to accommodate the press type electric generator, electric storage device and the electric heating layer.

The beneficial effects of the present effects are as follows: it comprises a sole main body in which a heating layer and a press type electric generator are arranged; the heating elements are distributed in the heating layer; the heating layer is connected with the press type electric generator; an intensive area is arranged on the heating layer; the distribution density of the heating elements in the intensive area is higher than that of the heating elements at other area of the heating layer; and the intensive area is arranged at a position where the heating layer corresponds to acupuncture points on the foot of a human body. The user treads on the press type electric generator while walking so as to convert the kinetic energy into the electric energy and power on the heating layer to heat the sole, thus to supply heat to maintain proper temperature for the feet of human body; at the same time, because the distribution density of the heating elements in the intensive area is higher than that of the heating elements at other area of the heating layer, so the generated heat is more concentrated to effectively supply heat for the parts of the acupuncture points on the foot of a human body directly corresponding to visceral organs of human body. The invention has good comprehensive warming function; the special requirement that the important parts of the foot of the human body are kept warm can be met; the distribution of the produced heat is reasonable; the application range is wide; and the electric power can be supplied by the sole itself.

### BRIEF DESCRIPTION OF THE DRAWINGS

The drawings are used for further description to the invention, but the embodiments in the figures do not limit the invention.

Fig.1 is the structural schematic diagram of the electric heating sole of the invention;

Fig.2 is the structural schematic diagram of the micro electric generator in the electric heating sole of the invention; and

Fig.3 is the structural schematic diagram of the embodiment 2 of the invention.

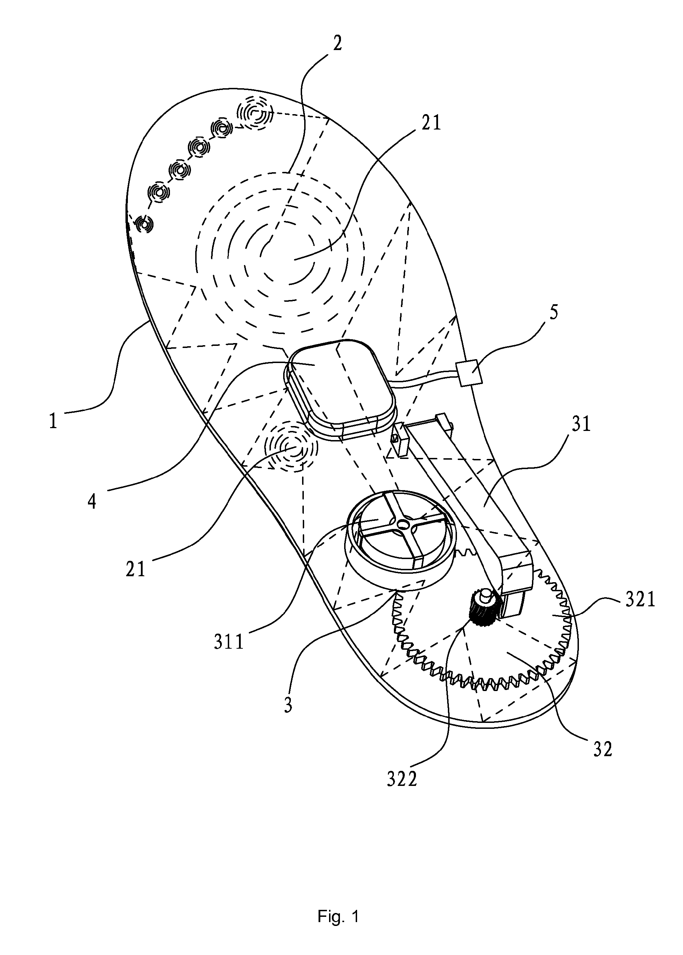

Figures 1 to 3 comprise:

| | |
|---|---|
| 1. Sole main body; | 2. Heating layer; |
| 3. Press type electric generator; | 4. Electric storage device; |
| 5. Control switch; | 21. Intensive area; |
| 31. Tread mechanism; | 32. Driving wheel; |
| 33. Micro electric generator; | 311. Electric element; |
| 321. First gearing; | 322. Second gearing; |
| 323. Third gearing; | 331. Ratchet wheel; |
| 332. Rotation disc; | 333. Detents; |
| 334. Detent blocks. | |

### DETAILED DESCRIPTION OF THE PREFERRED EMBODIMENTS

The following is the further description of the invention combining the drawings and embodiments.

Embodiment 1

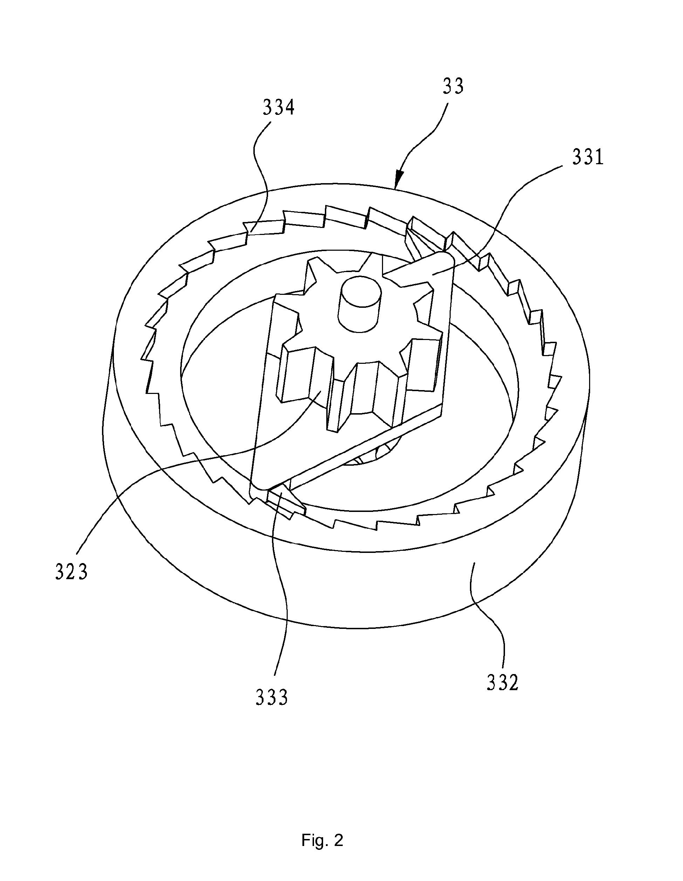

As shown in the Fig.1 and Fig.2, the invention comprises a sole main body 1 in which a heating layer 2 and a press type electric generator 3 are arranged; the heating elements are distributed in the heating layer 2; the heating layer 2 is connected with the press type electric generator 3; an intensive area 21 is arranged on the heating layer 2; the distribution density of the heating elements in the intensive area 21 is higher than that of the heating elements at other area of the heating layer 2; and the intensive area 21 is arranged at a position where the heating layer 2 corresponds to acupuncture points on the foot of a human body.

The heating layer 2 of the invention is powered by the power supply provided by the press type electric generator 3 to heat the sole, thus to supply heat to maintain proper temperature for the feet of human body; at the same time, because the distribution density of the heating elements in the intensive area 21 is higher than that of the heating elements at other area of the heating layer 2, so the generated heat is more concentrated to effectively supply heat for the parts of the acupuncture points on the foot of a human body directly corresponding to visceral organs of human body. The invention has good comprehensive warming function; the special requirement that the important parts of the foot of the human body are kept warm can be met; the distribution of the produced heat is reasonable; the application range is wide; and the electric power can be supplied by the sole itself.

The heating element can be resistance wire which is wound between two layers of heat-resistant insulating cloth or fixedly distributed on a base cloth to form the heating layer 2. The resistance wire is uniformly wound on other areas except the intensive area 21 and is intensively wound in the intensive area 21, so the distribution density of the resistance wire in the intensive area 21 is higher than that of the resistance wire at other areas. The heating element can also be electric heating band or other flake-like electric heating body. The electric heating bands are clamped between two heat-resistant insulating layers at intervals, or fixedly laid on a base to form the heating layer 2; electric heating bands are connected with one another in series or in parallel through power line; the electric heating bands are uniformly distributed at intervals at other areas except the intensive area 21, and are intensively distributed at the intensive area 21, so the distribution density of the electric heating bands in the intensive area 21 is higher than that of the electric heating bands at other areas. The resistance wire or flake-like electric heating band is common heating element applied to the electric heating sole, if only it can be powered with electricity to heat the sole.

The intensive area 21 is arranged at positions where the heating layer 2 corresponds to arch of foot, forefoot and toe of the human body where there are acupunctures. Since there are many acupunctures on foot that are related to visceral organs of human body, that is to say, keeping feet warm can benefit the health of visceral organs of human body. Specifically, the position of the intensive area can be set based on the human body constitution, living environment or gender. For example, for the user having higher requirement of keeping warm for brain and the five sense organs, the intensive area 21 can be set at the position on heating layer 2 corresponding to the acupunctures on toe; for the user having higher requirement of keeping warm for shoulder, heart, lung, liver, gallbladder, spleen, pancreas and stomach, the intensive area 21 can be set at the position on heating layer 2 corresponding to the acupunctures on the forefoot. Similarly, the intensive area 21 can be set at the position on heating layer 2 corresponding to the acupunctures on the arc of foot, so as to provide heat intensively to these parts and make the heat distribution more reasonable, thus to bring health benefits for the acupunctures on feet related to the parts of human body.

The press type electric generator 3 comprises a tread mechanism 31, and the sole main body is provided with a fixing block. One end of the tread mechanism 31 is in swing connection with the fixing block on the sole main body 1 through a connection axle which is sleeved with a spring, and the other end of the tread mechanism 31 is provided with arc-shaped spline which is connected with the driving wheel 32. And the driving wheel 32 comprises a first gearing 321 and a second gearing 322 that are coaxial and fixedly connected with each other, namely, they rotate at the same time; the first gearing 321 is engaged with the arc-shaped spline of the tread mechanism 31 and the second gearing 322 is connected with the micro electric generator 33.

Further, the micro electric generator 33 comprises a third gearing 323 that is engaged with the second gearing 322 of the driving wheel 32, and the third gearing 323 is fixedly connected with a ratchet wheel 331 which is provided with a rotation disc 332 on the top; electric generating elements 311 are arranged in the rotation disc 332; both sides of the ratchet wheel 331 are provided with detents 333 which can only rotate towards one direction with the other direction blocked by the ratchet wheel 331, and inner wall of the rotation disc 332 is provided with multiple detent blocks 334 to coordinates with the multiple detents 333.

When the user is walking, the tread mechanism is treaded down by foot of the user to drive the first gearing 321 to rotate; when the first gearing 321 is rotating, the second gearing 322 also rotates synchronously with it, and the first gearing 321 also drives the third gearing 323 to rotate; because the third gearing 323 is fixedly connected with the ratchet wheel 331 which is provided with a rotation disc 332 on the top and electric generating elements 311 are arranged in the rotation disc 332, the ratchet wheel 331 also rotates when the third gearing 323 rotates; and both sides of the ratchet wheel 331 are provided with detents 333, and inner wall of the rotation disc 332 is provided with multiple detent blocks 334 to coordinates with the multiple detents 333, so when the ratchet wheel 331 rotates, the detents 333 rotate to the direction blocked by the ratchet wheel 331; as a result, the detents 333 are blocked between two detent blocks 334 to rotate the rotation disc 332; because there are electric generating elements 311 arranged in the rotation disc 332, when the rotation disc 332 rotates, electric energy will be generated to power on the heating layer 2, and the voltage is not greater than 36V; when the user is walking and starting the next step, the tread mechanism 31 is released from foot and swings far away from the sole main body under effect of the spring, and the first gearing 321, the second gearing 322, the third gearing 323 and the ratchet wheel 331 are driven to rotate reversely; when the ratchet wheel 331 rotate reversely, the detents 333 rotate to the direction where the detents 333 are not blocked by the ratchet wheel 331 or blocked between two detent blocks 334 or contact with the rotation disc 332; at this time, the rotation disc 332 and the electric generating elements 311 are fixed and cannot generate electric energy; and this cycle repeats.

Embodiment 2

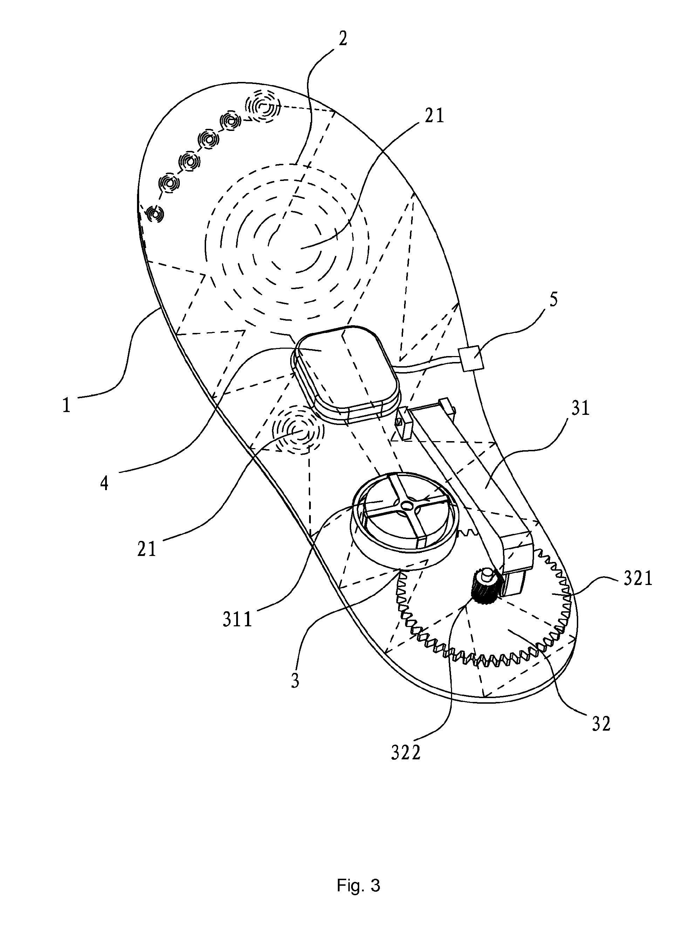

As shown in Fig.3, the difference from the embodiment 1 is that an electric storage device 4 is arranged between the micro electric generator 33 and the heating layer 2 in this embodiment. The electric storage device 4 is separately connected with the micro electric generator 33 and the heating layer 2 to store the electric energy output by the micro electric generator 33. When the user does not walk, the tread mechanism 31 cannot be treaded, so the micro electric generator 33 cannot generate electric energy. The redundant electric energy generated by the micro electric generator 33 while the user is walking can be stored and used for keeping feet warm when the user is not walking.

The electric storage device 4 is connected with a control switch 5 that is arranged outside of the sole main body 1, and the control switch 5 comprises an ON shift, temperature adjustment shift and an OFF shift. When the user is walking or needs electric energy, the control switch is turned on, so the electric energy can be provided to the heating layer and the redundant electric energy generated by the micro electric generator 33 can be stored. When the feet feel that the temperature is too high or too low, the temperature can be adjusted to the most comfortable degree through the temperature adjustment shift. When the invention is not used, the output of electric energy from the electric storage device can be stopped by pressing the OFF shift to avoid waste of energy.

The sole main body 1 is provided with an accommodation cavity to accommodate the press type electric generator 3, the electric storage device 4 and the electric heating layer, which facilitates dismounting and replacement of the whole device. Besides, the press type electric generator 3, the electric storage device 4 and the electric heating layer can be placed into three different accommodation cavities to facilitate dismounting and replacement of the single device.

Other parts of this embodiment are the same as that of the embodiment one.

The above embodiments are only preferable implementation methods of the present invention. Therefore, all changes and modifications based on the structure, feature and principle belonging to the patent application scope of present invention are claimed to be in the protective scope of the present invention.

## Claims

1. An electric heating sole, comprising a sole main body (1) in which a heating layer (2) distributed with heating elements is arranged, **characterized in that** a press type electric generator (3) is arranged in the sole main body (1), and the heating layer (2) is connected with the press type electric generator (3), an intensive area (21) is arranged on the heating layer (2), the distribution density of the heating elements in the intensive area (21) is higher than that of the heating elements at other area of the heating layer (2) and the intensive area (21) is arranged at a position where the heating layer (2) corresponds to acupuncture points on the foot of a human body.

2. The electric heating sole according to claim 1, **characterized in that** the intensive area (21) is arranged at positions where the heating layer (2) corresponds to arch of foot, forefoot and toe of the human body.

3. The electric heating sole according to claim 2, **characterized in that** the press type electric generator (3) includes a tread mechanism (31), one end of which is connected with a driving wheel (32) that is connected with a micro electric generator (33), and output end of the micro electric generator (33) is connected with the heating layer (2).

4. The electric heating sole according to any one of claims 1 to 3, **characterized in that** an electric storage device (4) is arranged between the micro electric generator (33) and the heating layer (2), and the electric storage device (4) is separately connected with the micro electric generator (33) and the heating layer (2).

5. The electric heating sole according to claim 3, **characterized in that** one end of the tread mechanism (31) is in swing connection with the sole main body (1) through a connection axle which is sleeved with a spring; and the other end of the tread mechanism (31) is provided with arc-shaped spline connected with the driving wheel (32).

6. The electric heating sole according to claim 5, **characterized in that** the driving wheel (32) comprises a first gearing (321) and a second gearing (322) that are coaxial and fixedly connected with each other; the first gearing (321) is engaged with the arc-shaped spline of the tread mechanism (31) and the second gearing (322) is connected with the micro electric generator (33).

7. The electric heating sole according to claim 6, **characterized in that** the micro electric generator (33) comprises a third gearing (323) that is engaged with the second gearing (322) of the driving wheel (32), and the third gearing (323) is fixedly connected with a ratchet wheel (331) which is provided with a rotation disc (332) on the top; electric generating elements are arranged in the rotation disc (332), both sides of the ratchet wheel (331) are provided with detents, and inner wall of the rotation disc (332) is provided with multiple detent blocks (334) to coordinates with the multiple detents (333).

8. The electric heating sole according to claim 4, **characterized in that** the electric storage device (4) is connected with a control switch (5) that is arranged outside of the sole main body.

9. The electric heating sole according to claim 8, wherein the control switch (5) comprises an ON shift, temperature adjustment shift and an OFF shift.

10. The electric heating sole according to claim 9, **characterized in that** the sole main body (1) is provided with an accommodation cavity to accommodate the press type electric (33) generator, electric storage device (4) and the electric heating layer (2).
